# EUROPEAN PATENT APPLICATION

(11) **EP 4 703 477 A1**
(43) Date of publication of application: **04.03.2026**
(21) Application number: 24797183.1
(22) Date of filing: 26.04.2024
(51) Int. Cl.: C12P 19/30, C12N 1/00, C12N 1/20, C12R 1/225

(54) **METHOD FOR PRODUCING YOGURT**

(30) Priority: 28.04.2023 JP 2023074942
(71) Applicant: Meiji Holdings Co., Ltd., Tokyo 104-0031 (JP)
(72) Inventor: OZAKI, Satoru, Hachioji-shi, Tokyo 192-0919 (JP); HONME, Yoshiko, Hachioji-shi, Tokyo 192-0919 (JP); MORIFUJI, Masashi, Hachioji-shi, Tokyo 192-0919 (JP)
(74) Representative: TBK
(86) International application number: PCT/JP2024/016459
(87) International publication number: WO 2024/225438

(57) **Abstract**

A method for producing yogurt, comprising a culturing step of culturing at least one NMN-producing lactic acid bacterium selected from the group consisting of Lactobacillus delbrueckii and Limosilactobacillus reuteri in an NAD-containing medium prepared by adding nicotinamide adenine dinucleotide to a milk component-containing medium, and obtaining a yogurt containing nicotinamide mononucleotide.

## Description

### [Technical Field]

The present invention relates to a method for producing yogurt, and more specifically, relates to a method for producing yogurt containing nicotinamide mononucleotide.

### [Background Art]

Nicotinamide mononucleotide (NMN) is a precursor of nicotinamide adenine dinucleotide (NAD) and is a substance that has been receiving particular attention in recent years.

For example, maintenance of the activity of sirtuins, which are called longevity genes, is considered important for the control of aging, but a decrease in the tissue concentration of NAD required for that activity accompanies getting older (aging); since this is considered a cause of aging, the administration or intake of NMN, which is a precursor of NAD, is said to bring about an anti-aging effect.

More specifically, for example, Mills Kathryn F. et al., Cell metabolism, 24.6, 2016, p. 795-806 (NPL 1) reports that administration of NMN to aged mice confirmed the suppression of obesity due to aging, the improvement of energy metabolism, and the improvement of insulin resistance, lipid metabolism, eye function, bone density, and immune function, and resulted in the suppression of changes in gene expression due to aging in skeletal muscle, fat, and liver. Furthermore, type II diabetes is a lifestyle-related disease caused in part by a decrease in glucose tolerance due to aging, but Jun Yoshino et al., Cell Metab., 2011 Oct 5, 14(4), p. 528-536 (NPL 2) reports that in mice induced with type II diabetes by high-fat diet administration, administration of NMN confirmed the improvement of impaired glucose tolerance and hepatic insulin sensitivity, and confirmed the recovery of gene expression related to oxidative stress, inflammatory response, and circadian rhythm.

In addition, for example, Luis Rajman et al., Cell Metab., 2018 Mar 6, 27(3), p. 529-547 (NPL 3) and Xiaonan Wang et al., Brain Research, Volume 1643, 15 July 2016, p. 1-9 (NPL 4) report that the administration of NMN improved liver function, kidney function, skeletal muscle function, and heart function, which had declined due to aging, as well as Alzheimer's type dementia. Furthermore, for example, Zhu et al., Signal Transduction and Targeted Therapy, 2017, e17017 (NPL 5) reports that the administration of NMN to aged mice improved constipation.

For that reason, supplements and the like for ingesting NMN have been commercially available in recent years. However, a method for easily producing an industrially sufficient amount of NMN has not yet been established, and because it is difficult to obtain raw materials, there was a problem of being expensive for daily intake.

As methods for producing NMN, in addition to production by chemical synthesis, a method of producing it using nicotinamide riboside (NR) as a raw material, a method of producing it with Escherichia coli, a method of producing it with yeast, and the like are known. Furthermore, International Publication No. WO 2022/138656 (PTL 1) describes a method for producing NMN that includes a step of reacting a specific lactic acid bacterium belonging to the genus Fructobacillus in a reaction solution with a pH of 4 to 10, and International Publication No. WO 2021/070829 (PTL 2) describes that a similar lactic acid bacterium produces NMN and NR.

### [Citation List]

### [Patent Literature]

[PTL 1] International Publication No. WO 2022/138656
[PTL 2] International Publication No. WO 2021/070829

### [Non Patent Literature]

[NPL 1] Mills Kathryn F. et al., Cell metabolism, 24.6, 2016, p. 795-806
[NPL 2] Jun Yoshino et al., Cell Metab., 2011 Oct 5, 14(4), p. 528-536
[NPL 3] Luis Rajman et al., Cell Metab., 2018 Mar 6, 27(3), p. 529-547
[NPL 4] Xiaonan Wang et al., Brain Research, Volume 1643, 15 July 2016, p. 1-9
[NPL 5] Zhu et al., Signal Transduction and Targeted Therapy, 2017, e17017

### [Summary of Invention]

### [Technical Problem]

Since lactic acid bacteria are microorganisms having a rich dietary history, if it were possible to produce NMN with lactic acid bacteria, and particularly to produce NMN-containing yogurt as yogurt with a long-standing dietary history, it would be desirable from a safety perspective. However, there was a problem in that it is still difficult to produce a sufficient amount of NMN with lactic acid bacteria by conventional methods.

The present invention was made in view of the problems of the above-described conventional art, and an object thereof is to provide a method for producing yogurt that enables the production of yogurt containing a sufficient amount of nicotinamide mononucleotide (NMN) using Lactobacillus delbrueckii and Limosilactobacillus reuteri.

### [Solution to Problem]

The present inventors conducted diligent research to achieve the above object, and first, when they searched for bacterial species that produce NMN from among many lactic acid bacteria, they found that NMN is produced, albeit at a relatively low concentration, in Lactobacillus delbrueckii and Limosilactobacillus reuteri. Next, as a result of conducting studies to improve the amount of NMN production by these lactic acid bacteria, they found that by adding NAD to a medium and culturing, the production amount of NMN significantly increases, and furthermore, they found that even among the above lactic acid bacteria, strains that did not produce NMN without the addition of NAD come to produce NMN. In addition, they also found that by adding NAD to a milk component-containing medium and culturing (fermenting), it is possible to produce yogurt containing a sufficient amount of NMN, and have thereby completed the present invention.

Aspects of the present invention obtained from such findings are as follows.
[1] A method for producing yogurt, comprising a culturing step of culturing at least one NMN-producing lactic acid bacterium selected from the group consisting of Lactobacillus delbrueckii and Limosilactobacillus reuteri in an NAD-containing medium prepared by adding nicotinamide adenine dinucleotide to a milk component-containing medium, and obtaining a yogurt containing nicotinamide mononucleotide.
[2] The method for producing yogurt according to [1], wherein a content of nicotinamide mononucleotide derived from the NAD-containing medium and the lactic acid bacterium in the yogurt is 5 ng/mL or more.
[3] The method for producing yogurt according to [1] or [2], wherein a content of milk components in the milk component-containing medium is 5 to 35 w/w% in terms of a content of non-fat milk solids.
[4] The method for producing yogurt according to any one of [1] to [3], wherein a temperature in the culturing is 25 to 55°C.
[5] The method for producing yogurt according to any one of [1] to [4], wherein a content of nicotinamide adenine dinucleotide in the NAD-containing medium is 1 × 10² to 1 × 10⁸ ng/mL relative to a total amount of the NAD-containing medium.
[6] The method for producing yogurt according to any one of [1] to [5], wherein the addition of nicotinamide adenine dinucleotide to the medium is the addition of Streptococcus thermophilus, which produces nicotinamide adenine dinucleotide in the medium.
[7] A method for producing nicotinamide mononucleotide, comprising a culturing step of culturing at least one NMN-producing lactic acid bacterium selected from the group consisting of Lactobacillus delbrueckii and Limosilactobacillus reuteri in an NAD-containing medium prepared by adding nicotinamide adenine dinucleotide to a medium.
[8] The method for producing nicotinamide mononucleotide according to [7], wherein the medium is a milk component-containing medium.
[9] The method for producing nicotinamide mononucleotide according to [8], wherein a content of milk components in the milk component-containing medium is 5 to 35 w/w% in terms of a content of non-fat milk solids.
[10] The method for producing nicotinamide mononucleotide according to any one of [7] to [9], wherein a temperature in the culturing is 25 to 55°C.
[11] The method for producing nicotinamide mononucleotide according to any one of [7] to [10], wherein a content of nicotinamide adenine dinucleotide in the NAD-containing medium is 1 × 10² to 1 × 10⁸ ng/mL relative to a total amount of the NAD-containing medium.
[12] The method for producing nicotinamide mononucleotide according to any one of [7] to [11], wherein the addition of the nicotinamide adenine dinucleotide to the medium is the addition of at least one selected from the group consisting of Streptococcus thermophilus, which produces nicotinamide adenine dinucleotide in the medium, and a bacterium belonging to the genus Bifidobacterium.
[13] A method for improving a nicotinamide mononucleotide production amount, comprising a step of adding nicotinamide adenine dinucleotide to a medium for culturing at least one NMN-producing lactic acid bacterium selected from the group consisting of Lactobacillus delbrueckii and Limosilactobacillus reuteri to make an NAD-containing medium.
[14] The method for improving a nicotinamide mononucleotide production amount according to [13], wherein the medium is a milk component-containing medium.
[15] The method for improving a nicotinamide mononucleotide production amount according to [14], wherein a content of milk components in the milk component-containing medium is 5 to 35 w/w% in terms of a content of non-fat milk solids.
[16] The method for improving a nicotinamide mononucleotide production amount according to any one of [13] to [15], wherein the culturing is performed at a temperature of 25 to 55°C.
[17] The method for improving a nicotinamide mononucleotide production amount according to any one of [13] to [16], wherein an addition amount of the nicotinamide adenine dinucleotide is an amount such that a content thereof is 1 × 10² to 1 × 10⁸ ng/mL relative to a total amount of the NAD-containing medium.
[18] The method for improving a nicotinamide mononucleotide production amount according to any one of [13] to [17], wherein the addition of the nicotinamide adenine dinucleotide to the medium is the addition of at least one selected from the group consisting of Streptococcus thermophilus, which produces nicotinamide adenine dinucleotide in the medium, and a bacterium belonging to the genus Bifidobacterium.

### [Advantageous Effects of Invention]

According to the present invention, it becomes possible to provide a method for producing yogurt that enables the production of yogurt containing a sufficient amount of nicotinamide mononucleotide (NMN) using Lactobacillus delbrueckii and Limosilactobacillus reuteri. Furthermore, according to the present invention, it also becomes possible to provide an NMN production method capable of producing a sufficient amount of NMN, and an NMN production amount improvement method capable of improving the amount of NMN production by the lactic acid bacteria.

### [Brief Description of Drawings]

[Fig. 1] Fig. 1 is a graph showing the NMN concentration (ng/mL) in SMY medium after culturing Lactobacillus delbrueckii, Limosilactobacillus reuteri, and Streptococcus thermophilus shown in Table 1 in the SMY medium.
[Fig. 2] Fig. 2 is a graph showing the NMN concentration (ng/mL) in SMY+NAD medium after culturing Lactobacillus delbrueckii, Limosilactobacillus reuteri, and Streptococcus thermophilus shown in Table 1 in the SMY+NAD medium.
[Fig. 3] Fig. 3 is a graph showing the NMN concentration (ng/mL) in SMY medium after culturing the combinations of Limosilactobacillus reuteri and Streptococcus thermophilus shown in Table 3 in the SMY medium.
[Fig. 4] Fig. 4 is a graph showing the NMN concentration (ng/mL) in SMY medium after culturing the combinations of Lactobacillus delbrueckii and Streptococcus thermophilus shown in Table 4 in the SMY medium.
[Fig. 5] Fig. 5 is a graph showing the NMN concentration (ng/mL) in SMY medium after culturing a combination of Lr MHD202317 and St MHD202312 at 37°C or 43°C in SMY medium of each milk component concentration.
[Fig. 6] Fig. 6 is a graph showing the NMN concentration (ng/mL) in SMY medium after culturing a combination of Lb MHD202324 and St MHD202312 at 37°C or 43°C in SMY medium of each milk component concentration.

### [Description of Embodiments]

Hereinafter, the present invention will be described in detail in accordance with preferred embodiments thereof.

The present invention provides a method for producing nicotinamide mononucleotide (in this specification, sometimes referred to as "NMN production method"), comprising a culturing step of culturing at least one NMN-producing lactic acid bacterium selected from the group consisting of Lactobacillus delbrueckii and Limosilactobacillus reuteri in an NAD-containing medium prepared by adding nicotinamide adenine dinucleotide to a medium.

The present invention also provides a method for improving a nicotinamide mononucleotide production amount (in this specification, sometimes referred to as "NMN production amount improvement method"), comprising a step of adding nicotinamide adenine dinucleotide to a medium for culturing at least one NMN-producing lactic acid bacterium selected from the group consisting of Lactobacillus delbrueckii and Limosilactobacillus reuteri to make an NAD-containing medium.

Furthermore, the present invention also provides a method for producing yogurt (in this specification, sometimes referred to as "yogurt production method"), comprising a culturing step of, by using a milk component-containing medium as the medium, culturing at least one NMN-producing lactic acid bacterium selected from the group consisting of Lactobacillus delbrueckii and Limosilactobacillus reuteri in an NAD-containing medium prepared by adding nicotinamide adenine dinucleotide to the milk component-containing medium, and obtaining a yogurt containing nicotinamide mononucleotide.

### (NMN)

"Nicotinamide mononucleotide" indicates β-nicotinamide mononucleotide, and is also abbreviated as "NMN." β-Nicotinamide mononucleotide is an intermediate metabolite when nicotinamide and nicotinamide riboside (NR) are converted to nicotinamide adenine dinucleotide (NAD) in vivo. The present inventors found that NMN is produced by the below-described Lactobacillus delbrueckii and Limosilactobacillus reuteri, and that the addition of nicotinamide adenine dinucleotide enhances the production amount of such NMN or expresses the production ability, and has an excellent NMN production ability-improving action.

### (NAD)

"Nicotinamide adenine dinucleotide" is a substance consisting of NMN and adenosine monophosphate (AMP), and is known to play a central role as a redox coenzyme in dehydrogenation reactions in vivo. In the present invention, nicotinamide adenine dinucleotide may be an oxidized form (NAD⁺), a reduced form (NADH), or a mixture thereof. In this specification, these are sometimes collectively referred to as "NAD."

### (NMN-producing lactic acid bacterium)

In the present invention, the lactic acid bacteria that produce NMN, that is, have NMN-producing ability, are Lactobacillus delbrueckii and Limosilactobacillus reuteri. These may be only one species or a combination of both two species. In this specification, at least one lactic acid bacterium having NMN-producing ability selected from the group consisting of Lactobacillus delbrueckii and Limosilactobacillus reuteri is sometimes collectively referred to as an "NMN-producing lactic acid bacterium" (or "NMN-producing lactic acid bacteria").

### [Lactobacillus delbrueckii]

Lactobacillus delbrueckii is a Lactobacillus genus lactic acid bacterium belonging to the family Lactobacillaceae. Lactobacillus delbrueckii according to the present invention also includes its subspecies, Lactobacillus delbrueckii ssp.

Examples of the Lactobacillus delbrueckii subspecies include Lactobacillus delbrueckii ssp. bulgaricus, Lactobacillus delbrueckii ssp. delbrueckii, Lactobacillus delbrueckii ssp. lactis, Lactobacillus delbrueckii ssp. indicus, Lactobacillus delbrueckii ssp. sunkii, and Lactobacillus delbrueckii ssp. jakobsenii.

As for Lactobacillus delbrueckii according to the present invention, it may be only one of them or a combination of two or more, but among them, Lactobacillus delbrueckii ssp. bulgaricus (also called "Bulgarian bacterium") is particularly preferable.

Lactobacillus delbrueckii according to the present invention is not particularly limited as long as it has NMN-producing ability, that is, in the present invention, it is capable of producing NMN in the below-described NAD-containing medium; for example, when 1 × 10⁴ to 1 × 10⁸ cfu/g is inoculated into SMY+NAD medium (10 w/w% skim milk powder, 0.1 w/w% yeast extract, 5,000 ng/mL NAD⁺) and anaerobically cultured at 37°C for 12 hours, one that produces 2 ng/mL or more, more preferably 10 ng/mL or more, still more preferably 30 ng/mL or more, and even more preferably 50 ng/mL or more of NMN in the SMY+NAD medium after culturing can be selected. In the present invention, NMN can be detected and measured, for example, by LC-MS/MS analysis, and as for the conditions, for example, the conditions described in the Examples below can be adopted.

Lactobacillus delbrueckii according to the present invention is not particularly limited, but more specific examples include Lactobacillus delbrueckii ssp. bulgaricus JCM 1002^{T} strain (Lb JCM1002T strain) and the like, and the strain may be a subculture thereof, or an artificial mutant, natural mutant, genetically modified strain, derivative strain, or the like of the strain or a subculture thereof, within a range that does not impair the effects of the present invention. In this specification, strains indicated by a JCM strain number are strains available from the Japan Collection of Microorganisms (JCM), RIKEN BioResource Research Center (http://jcm.brc.riken.jp/ja/). Furthermore, Lactobacillus delbrueckii according to the present invention may be a lactic acid bacterium isolated from commercially available fermented milk with a known agar medium for the genus Lactobacillus (e.g., MRS agar, Plate Count Agar with BCP).

### [Limosilactobacillus reuteri]

Limosilactobacillus reuteri is a Limosilactobacillus genus lactic acid bacterium belonging to the family Lactobacillaceae. As for Limosilactobacillus reuteri according to the present invention, it may be only one of them or a combination of two or more.

Limosilactobacillus reuteri according to the present invention is not particularly limited as long as it has NMN-producing ability, that is, in the present invention, it is capable of producing NMN in the below-described NAD-containing medium; for example, when 1 × 10⁴ to 1 × 10⁸ cfu/g is inoculated into SMY+NAD medium (10 w/w% skim milk powder, 0.1 w/w% yeast extract, 5,000 ng/mL NAD⁺) and anaerobically cultured at 37°C for 12 hours, one that produces 10 ng/mL or more, more preferably 100 ng/mL or more, and still more preferably 200 ng/mL or more of NMN in the SMY+NAD medium after culturing can be selected.

Limosilactobacillus reuteri according to the present invention is not particularly limited, but more specific examples include Limosilactobacillus reuteri JCM 1112^{T} strain (Lr JCM1112T strain) and the like, and the strain may be a subculture thereof, or an artificial mutant, natural mutant, genetically modified strain, derivative strain, or the like of the strain or a subculture thereof, within a range that does not impair the effects of the present invention. Furthermore, Limosilactobacillus reuteri according to the present invention may be a lactic acid bacterium isolated from commercially available fermented milk with a known agar medium for the genus Lactobacillus (e.g., MRS agar, Plate Count Agar with BCP).

### (Medium)

In the present invention, when simply referred to as "medium," it indicates one that does not contain NAD, that is, a medium before the addition of NAD. The medium according to the present invention is not particularly limited as long as it is a medium in which the NMN-producing lactic acid bacterium can be cultured, and examples include MRS medium, GYP medium, and a milk component-containing medium. Among these, a milk component-containing medium is particularly preferable in the NMN production method and the NMN production amount improvement method of the present invention, and a milk component-containing medium is necessary in the yogurt production method of the present invention. In the present invention, when simply referred to as a "milk component-containing medium," it indicates a medium that contains milk components and does not contain NAD.

In the present invention, the milk components indicate milk and milk products that contain at least non-fat milk solids (that is, in which non-fat milk solids are detectable as the remaining component after subtracting the fat content from the total milk solids), and examples include raw milk (e.g., milk from cows, water buffalo, sheep, goats, etc.), whey, and processed products thereof (e.g., skim milk, skim milk powder, concentrated skim milk, whole milk, whole milk powder, concentrated whole milk, sterilized milk, condensed milk, whey powder, buttermilk, butter, cream, cheese, whey protein concentrate (WPC), whey protein isolate (WPI), α-lactalbumin (α-La), β-lactoglobulin (β-Lg)), and the like, and these may be one species or a combination of two or more.

The milk component-containing medium may consist only of the milk components or may contain the milk components and other components. Examples of the other components include water (e.g., ultrapure water), yeast extract, saccharides, sugar alcohols, minerals, vitamins, proteins, peptides, amino acids, organic acids, pH adjusters, starch and modified starch, dietary fiber, fruits/vegetables and processed products thereof, meat, animal and plant crude drug extracts, naturally derived polymers (collagen, hyaluronic acid, chondroitin, etc.), oils and fats, thickeners, emulsifiers, solvents, surfactants, gelling agents, stabilizers, buffers, suspending agents, viscosity agents, excipients, disintegrants, binders, fluidizers, preservatives, coloring agents, flavoring agents, correctives, sweeteners, and the like, and these may be one species or a combination of two or more. In the present invention, the yeast extract is an extract of the contents of yeast, and as the yeast, for example, Saccharomyces cerevisiae is preferable. The yeast extract contains amino acids, peptides, nucleic acids, minerals, etc., and although their composition differs depending on the type of yeast, culture conditions, extraction conditions, etc., any yeast extract can be used without particular limitation.

The content of the milk components in the milk component-containing medium is preferably 5 to 35 w/w%, more preferably 10 to 23 w/w%, still more preferably 15 to 22 w/w%, and even more preferably 16 to 21 w/w%, relative to the total amount of the milk component-containing medium (not including NAD, the same applies hereinafter), in terms of the content of non-fat milk solids. The content of the non-fat milk solids can be measured by the method described in Japan's "Ministerial Ordinance on Milk and Milk Products Concerning Compositional Standards, etc. (hereinafter, sometimes referred to as 'Milk Ordinance')."

When the milk component-containing medium contains the yeast extract, its content is preferably 0.001 to 1 w/w%, and more preferably 0.001 to 0.1 w/w%, relative to the total amount of the milk component-containing medium. The pH of such a milk component-containing medium is usually 6 to 7.

When performing the below-described activation culture, the medium used for the second and subsequent activation cultures and the culturing step may contain part or all of the culture solution after the previous culture. It is preferable that the media are each subjected to a sterilization treatment or pasteurization treatment.

### (NAD-containing medium)

The medium used in the culturing step according to the present invention is a medium to which NAD has been added to the above-described medium (preferably a milk component-containing medium; in the yogurt production method, a milk component-containing medium) (in this specification, sometimes referred to as "NAD-containing medium"). In the NMN production amount improvement method of the present invention, NAD is added to the medium to make the NAD-containing medium. In the NAD-containing medium, the content of NAD is preferably 1 × 10² to 1 × 10⁸ ng/mL, more preferably 1 × 10³ to 1 × 10⁸ ng/mL, and still more preferably 1 × 10³ to 1 × 10⁷ ng/mL, in terms of NAD⁺, relative to the total amount of the NAD-containing medium.

In the present invention, the method for adding NAD to the medium may be a method of preparing the NAD-containing medium by adding NAD⁺ or NADH itself to the medium, but as another aspect, for example, it may be a method of adding an NAD-producing bacterium (preferably, the below-described NAD-producing Streptococcus thermophilus) that produces nicotinamide adenine dinucleotide in the medium to the medium and performing culturing, thereby producing NAD in the medium to make the NAD-containing medium. In this case, the NMN-producing lactic acid bacterium and the NAD-producing bacterium may both be inoculated into the medium simultaneously, or the NAD-producing bacterium may be inoculated first. When the NAD-producing bacterium is inoculated first, the content of NAD in the NAD-containing medium can be adjusted in advance to the content listed above by first culturing the NAD-producing bacterium in the medium. On the other hand, when both are inoculated simultaneously, it is preferable that the content of NAD in the NAD-containing medium reaches the content listed above within 12 hours, preferably within 3 hours, from the start of culturing.

### [NAD-producing bacterium]

Examples of the NAD-producing bacterium according to the present invention include Streptococcus thermophilus, which produces nicotinamide adenine dinucleotide in the medium, and a bacterium belonging to the genus Bifidobacterium, and these may be one of these species or both species.

### - Streptococcus thermophilus

Streptococcus thermophilus is a streptococcus also called thermophilus bacterium, and is a lactic acid bacterium capable of producing lactic acid from lactose. As for Streptococcus thermophilus according to the present invention, it may be only one species or a combination of two or more.

Streptococcus thermophilus according to the present invention is not particularly limited as long as it has NAD-producing ability, that is, in the present invention, it is capable of producing NAD in the medium (NAD-producing Streptococcus thermophilus); for example, when 1 × 10⁶ to 1 × 10⁸ cfu/g is inoculated into SMY medium (10 w/w% skim milk powder, 0.1 w/w% yeast extract) and anaerobically cultured at 37°C for 12 hours, one that produces 1000 ng/mL or more, and more preferably 3000 ng/mL or more of NAD⁺ in the SMY medium after culturing can be selected. In the present invention, the amount of NAD⁺ can be measured, for example, by LC-MS/MS analysis, and as for the conditions, for example, the conditions described in the Examples below can be adopted. Any Streptococcus thermophilus usually satisfies the above conditions.

More specific examples of Streptococcus thermophilus according to the present invention include, but are not limited to, a bacterial strain such as Streptococcus thermophilus specified by Accession Number NITE BP-02875 (hereinafter, sometimes referred to as "St OLS4496 strain"). The St OLS4496 strain is deposited as follows: (1) Identification: Streptococcus thermophilus OLS4496; (2) Accession Number: NITE BP-02875; (3) Date of Deposit: February 5, 2019; and (4) Depositary Institution: NITE Patent Microorganisms Depositary (NPMD), National Institute of Technology and Evaluation (Room 122, 2-5-8 Kazusakamatari, Kisarazu-shi, Chiba 292-0818, Japan). The St OLS4496 strain may be a subculture of the strain, or an artificial mutant, natural mutant, genetically modified strain, derivative strain, or the like of the strain or a subculture thereof, within a range that does not impair the effects of the present invention.

### - Bacterium belonging to the genus Bifidobacterium

Other examples of the NAD-producing bacterium include a bacterium belonging to the genus Bifidobacterium. More specific examples of the bacterium belonging to the genus Bifidobacterium include Bifidobacterium breve JCM1192^{T}, Bifidobacterium longum JCM1217^{T}, Bifidobacterium adolescentis JCM1275^{T}, and Bifidobacterium pseudolongum JCM1205^{T}, and these strains may be subcultures thereof, or an artificial mutant, natural mutant, genetically modified strain, derivative strain, or the like of the strain or a subculture thereof, within a range that does not impair the effects of the present invention. It has been confirmed that these bacteria, when an activation culture solution is inoculated at 1 v/v% into GAM bouillon (Nissui Pharmaceutical Co., Ltd., #05422) and anaerobically cultured at 37°C for 24 hours, respectively produce 1178, 237, 945, and 947 ng/mL of NAD⁺ in the GAM bouillon after culturing. Furthermore, since other multiple strains of Bifidobacterium also produce 276 to 1390 ng/mL of NAD⁺ in a similar culture, it can be said that any bacterial strain belonging to the genus Bifidobacterium usually has NAD-producing ability.

### (Culturing step)

In the NMN production method, the NMN production amount improvement method, and the yogurt production method of the present invention, the NMN-producing lactic acid bacterium is cultured in the NAD-containing medium. In the yogurt production method of the present invention, culturing the NMN-producing lactic acid bacterium in the milk component-containing medium (NAD-containing) is synonymous with fermenting the milk component-containing medium with the NMN-producing lactic acid bacterium.

### [Activation culture]

It is preferable that the NMN-producing lactic acid bacterium and, if necessary, the NAD-producing bacterium to be subjected to the culturing according to the present invention are each subjected to an activation culture before the below-described culturing, for example, after low-temperature storage (e.g., 10°C or lower) or frozen storage. As for such an activation culture, for example, it is preferable to perform culturing in the medium (preferably a milk component-containing medium) at 37°C under anaerobic conditions for 4 to 40 hours. At this time, the amount of bacteria to be subjected to the activation culture is not particularly limited, but it is preferably an amount such that each bacterial amount is 1 platinum loopful/5 mL relative to the medium, and more specifically, it is preferably an amount such that the viable cell count is 1 × 10² to 2 × 10⁸ cfu/g, and more preferably 1 × 10⁴ to 1 × 10⁶ cfu/g. In the present invention, the measurement of the viable cell count can be measured, for example, by spreading a suitably diluted lactic acid bacterium-containing liquid on a suitable agar medium, culturing, and counting the number of colonies that appear.

The method of the activation culture is not particularly limited, and examples include static culture under conditions known as anaerobic culture conditions for conventional lactic acid bacteria. As for the anaerobic culture conditions, for example, culturing under a nitrogen aeration condition or culturing using a gas concentration adjuster (e.g., AnaeroPack (registered trademark, the same applies hereinafter) manufactured by Mitsubishi Gas Chemical Company, Inc.) can be adopted. The activation culture may be repeated multiple times (preferably twice).

### [Culturing]

In the culturing step according to the present invention, the NMN-producing lactic acid bacterium (or the NMN-producing lactic acid bacterium after the activation culture) is inoculated into the NAD-containing medium and cultured.

The inoculation amount of the NMN-producing lactic acid bacterium into the NAD-containing medium (the total if there are two or more species of NMN-producing lactic acid bacteria, the same applies hereinafter) is preferably an amount such that the viable cell count is 1 × 10⁴ to 1 × 10⁹ cfu/g, more preferably 1 × 10⁵ to 1 × 10⁹ cfu/g, and still more preferably 1 × 10⁶ to 1 × 10⁸ cfu/g, relative to the NAD-containing medium.

When Streptococcus thermophilus is added as the NAD-producing bacterium as a method for adding NAD to the medium, more specifically, as the culturing step according to the present invention, the NMN-producing lactic acid bacterium (or the NMN-producing lactic acid bacterium after the activation culture) and Streptococcus thermophilus (or Streptococcus thermophilus after the activation culture) are inoculated into the medium and cultured. The inoculation of Streptococcus thermophilus may be simultaneous with the inoculation of the NMN-producing lactic acid bacterium as described above, or it may be earlier.

When Streptococcus thermophilus is added as the method for adding NAD to the medium, the inoculation amount of the NMN-producing lactic acid bacterium into the medium (the total if there are two or more species of NMN-producing lactic acid bacteria, the same applies hereinafter) is the same as above. The inoculation amount of Streptococcus thermophilus is not particularly limited as long as the NAD content in the resulting NAD-containing medium satisfies the above conditions, but for example, it is preferably an amount such that the viable cell count is 1 × 10⁴ to 1 × 10⁹ cfu/g, more preferably 1 × 10⁵ to 1 × 10⁹ cfu/g, and still more preferably 1 × 10⁶ to 1 × 10⁸ cfu/g, relative to the medium.

The culturing conditions in the culturing step can be appropriately selected according to the growth conditions of each added NMN-producing lactic acid bacterium or NAD-producing bacterium, the amount of the NAD-containing medium or medium, and the like, and are not particularly limited, but the culture temperature is preferably 25 to 55°C, more preferably 30 to 46°C, still more preferably 35 to 45°C, and even more preferably 38 to 44°C.

As for other conditions in the culturing step, conditions known as culture conditions for conventional lactic acid bacteria or fermentation conditions for yogurt can be appropriately adopted, and either aerobic or anaerobic conditions may be used, and either static culture or shaking culture may be used. The culture time is usually 2 to 24 hours, more preferably 3 to 12 hours, and still more preferably 3 to 6 hours. Furthermore, it is preferable to culture until the pH of the culture solution (NAD-containing medium) becomes 5.5 or lower, and more preferable to culture until the pH becomes 4.6 or lower.

### (NMN-containing composition)

By the culturing step, NMN is produced in the NAD-containing medium, so that a culture containing NMN can be obtained after the culturing. In the yogurt production method of the present invention, by the culturing step, the milk component-containing medium is fermented and NMN is produced, so that a yogurt containing NMN can be obtained as the culture after the culturing (fermentation). The culture (including yogurt, the same applies hereinafter) contains NMN, the NMN-producing lactic acid bacterium, components derived from the NAD-containing medium, and, if necessary, Streptococcus thermophilus.

The content of NMN in the culture is not particularly limited, but according to the production method of the present invention, it can be, for example, 2 ng/mL or more, and more preferably 5 ng/mL or more, or 10 ng/mL or more; it can be still more preferably 15 ng/mL or more, and even more preferably 100 to 1 × 10⁷ ng/mL. The NMN contained in the culture (preferably yogurt) may be one that is separately added to the culture or the NAD-containing medium, but in the present invention, it can be, and is preferably, NMN excluding such added NMN, that is, NMN produced derived from the NAD-containing medium and the NMN-producing lactic acid bacterium. Alternatively, according to the NMN production amount improvement method of the present invention, the amount of NMN produced by the NMN-producing lactic acid bacterium can be made 1 ng/mL or more from 0 ng/mL when cultured in a medium without the addition of NAD, or it can be made 2 times or more, more preferably 5 times or more or 10 times or more, and still more preferably 15 times or more, compared to when cultured in a medium without the addition of NAD.

In the culture, the content of the NMN-producing lactic acid bacterium (viable cell content; if two or more species, the total amount; the same applies hereinafter) is preferably 1 × 10⁶ to 1 × 10¹¹ cfu/g, more preferably 5 × 10⁶ to 5 × 10¹⁰ cfu/g, and still more preferably 1 × 10⁷ to 1 × 10¹⁰ cfu/g, relative to the total mass of the culture.

When the culture contains Streptococcus thermophilus, its content (viable cell content, the same applies hereinafter) is preferably 1 × 10⁶ to 1 × 10¹¹ cfu/g, more preferably 5 × 10⁶ to 5 × 10¹⁰ cfu/g, and still more preferably 1 × 10⁷ to 1 × 10¹⁰ cfu/g, relative to the total mass of the culture.

From the culture obtained by the culturing step according to the present invention, NMN may be purified by a known method or a method analogous thereto. It may also be used as is, or by concentrating, diluting, drying, freezing, crushing, partially purifying, processing, etc., to form an NMN-containing composition.

As for the form of the NMN-containing composition, it can be, for example, a pharmaceutical composition, a quasi-drug composition, a food or beverage composition, or a feed composition, depending on the purpose, subject, method, dose, etc. of administering or ingesting the composition.

The pharmaceutical composition and the quasi-drug composition can be, for example, a formulation, and the form thereof is not particularly limited, but examples include solid preparations such as tablets, pills, granules, powders, and capsules; liquid preparations such as general liquids, suspensions, emulsions, and syrups; jelly preparations; enteral preparations; and external preparations such as suppositories. The formulation can be produced, for example, by adding a formulation auxiliary agent or other components to the culture (or a concentrate, diluted product, dried product, frozen product, crushed product, partially purified product, etc. thereof) by a known method or a method analogous thereto.

Examples of the formulation auxiliary agent include solvents, dispersants, emulsifiers, thickeners, thickening stabilizers, gelling agents, surfactants, buffers, stabilizers, excipients, binders, disintegrants, lubricants, correctives, solubilizing agents, suspending agents, coating agents, carriers (solid carriers, liquid carriers such as water), preservatives, flavoring agents, coloring agents, and pH adjusters, and these may be one species or a combination of two or more.

Examples of the other components include water, lipids, minerals, vitamins, proteins, peptides, amino acids, organic acids, other lactic acid bacteria or yeast and their processed products (crushed products, heat-treated products, etc.) or fermented products, and these may be one species or a combination of two or more.

The form of the food or beverage composition is not particularly limited, and examples include solid forms like bars, liquid forms like beverages and liquid foods, paste-like forms, semi-liquid forms, gel-like (jelly-like) forms, gel-like oils and fats (semi-solid oils and fats), and powder forms. The food or beverage composition can also be ingested by oral/enteral nutrition patients, the elderly, infants, etc., as liquid foods, powdered liquid foods, nutritional pastes, oral/enteral nutrients, beverages, gel-like foods, and the like.

Examples of the food or beverage composition are not particularly limited, but include, for example, beverages (teas, carbonated drinks, cocoa, coffee, lactic acid bacteria beverages, soy milk beverages, fruit/vegetable juice beverages, soft drinks, nutritional drinks, alcoholic beverages, etc.), processed foods (chocolate, gum, gummies, jellies, baked goods (bread, cakes, cookies, biscuits, etc.), candy, etc.), milk products (formulated milk powder (powdered milk, etc.), modified milk, milk beverages, ice cream, yogurt, cheese, cream, butter, margarine, condensed milk, etc.), seasonings (sauces, soups, dressings, mayonnaise, mayonnaise-type seasonings, cream, etc.), supplements, edible oils, and functional edible oils and fats. Such a food or beverage composition can be produced, for example, by a method of blending the culture into existing food or beverage products or their raw materials or intermediate products in the manufacturing process. The culture blended in this case may be in the form of the pharmaceutical composition or the quasi-drug composition.

As another aspect of the food or beverage composition, as described above, the culture (yogurt) obtained by the culturing step according to the yogurt production method of the present invention can be used as is, or by e.g. concentrating, diluting, drying, or freezing it, to make yogurt, which is a fermented milk.

Examples of the yogurt include fermented milk that meets the standards for "fermented milk" under the Milk Ordinance (more specifically, one with a non-fat milk solids content of 8.0% or more and a lactic acid bacteria count (the total count of the NMN-producing lactic acid bacteria and, if necessary, Streptococcus thermophilus; the same applies hereinafter) of 10 million/mL or more). The yogurt also includes those that meet the standards for "milk product lactic acid bacteria beverages" under the Milk Ordinance (more specifically, one with a non-fat milk solids content of 3.0% or more and a lactic acid bacteria count of 10 million/mL or more); and those that meet the standards for "lactic acid bacteria beverages" (more specifically, one with a non-fat milk solids content of less than 3.0% and a lactic acid bacteria count of 1 million/mL or more). The lactic acid bacteria count is the viable cell count measured by the inspection method specified in the Milk Ordinance.

The yogurt may further contain various components that can be included in food and beverage products. The various components are not particularly limited, but examples include saccharides, sugar alcohols, minerals, vitamins, proteins, peptides, amino acids, organic acids, pH adjusters, starch and modified starch, dietary fiber, fruits/vegetables and processed products thereof, animal and plant crude drug extracts, naturally derived polymers (collagen, hyaluronic acid, chondroitin, etc.), oils and fats, thickeners, emulsifiers, solvents, surfactants, gelling agents, stabilizers, buffers, suspending agents, viscosity agents, excipients, disintegrants, binders, fluidizers, preservatives, coloring agents, flavoring agents, correctives, sweeteners, and the like, and these may be one species or a combination of two or more.

Specific examples of such yogurt include set-type yogurt (solid fermented milk) such as plain yogurt, soft-type yogurt (paste-like fermented milk), and drink-type yogurt (liquid fermented milk), and it may be a frozen yogurt using these as materials. The yogurt can also be used as a material for fermented foods such as cheese, fermented cream, fermented butter, and kefir.

The food or beverage composition may be, for example, a general food, a health food, a functional food, a food with health claims (e.g., foods for specified health uses, foods with nutrient function claims, dietary supplements, foods with function claims, etc.), a food for special dietary uses (e.g., foods for infants, foods for pregnant and lactating women, foods for sick people, etc.), a medical food (a food prescribed under a physician's supervision as defined by the U.S. Food and Drug Administration (FDA) and the Orphan Drug Act), a therapeutic diet (one that serves a therapeutic purpose and is prepared based on a menu created by a nutritionist or the like according to a doctor's diet prescription), or a diet therapy food. The food or beverage composition may also display the actions and effects brought about by NMN in the product (for example, prevention or improvement of diseases due to aging, prevention or improvement of constipation, or prevention or improvement of inflammatory bowel disease).

Examples of the feed composition include those obtained by appropriately modifying the above food or beverage composition according to the purpose, subject, method, dose, etc. of administering or ingesting the feed composition.

The NMN-containing composition, by containing NMN as an active ingredient, can improve the NMN concentration in the body of a subject, and can also improve the production of NAD, which uses such NMN as a precursor, and furthermore its metabolites (nicotinamide, nicotinic acid, NAMN, NAAD, etc.), thereby improving their in vivo concentrations. Therefore, the NMN-containing composition can be used for improving NMN concentration and NAD concentration in vivo.

Furthermore, the NMN-containing composition, by improving the in vivo NMN concentration and NAD concentration, can also be used for the prevention or improvement of various diseases and symptoms that are reported to be prevented or improved by the administration or intake of NMN or its metabolite NAD, such as diseases due to aging (NPLs 1-4), constipation (NPL 5), and inflammatory bowel disease (Navarro et al., Br J Pharmacol., 2022, 179, p. 1839-1856), and is preferably used for the prevention or improvement of diseases due to aging, for the prevention or improvement of constipation, or for the prevention or improvement of inflammatory bowel disease.

Examples of the diseases due to aging include various diseases that are reported to be caused by aging (preferably 40 years or older, more preferably 50 years or older, still more preferably 60 years or older in humans), for example, obesity, decreased insulin resistance, decreased lipid metabolism ability, decreased eye function, decreased bone density, decreased immune function, decreased glucose tolerance, increased oxidative stress, inflammation, disruption of circadian rhythm, decreased liver function, decreased kidney function, decreased skeletal muscle function, decreased heart function, Alzheimer's type dementia, and decreased central nervous system function.

Examples of the subject include humans or non-human mammals, and examples of the non-human mammals include mice, sheep, cows, pigs, horses, monkeys, dogs, cats, and rabbits.

The NMN-containing composition can be administered to the subject as is, or more preferably as each of the compositions described above, and is preferably administered orally or enterally to the subject. In this specification, oral administration also includes the intake of the food or beverage compositions, the feed compositions, and the like.

The dosage or intake amount (hereinafter, also sometimes referred to as "dose") of the NMN-containing composition can be appropriately determined in each individual case, considering the form of the composition; the purpose and method of administration or intake; and the species, age, body weight, sex, degree of symptoms, and the like of the subject. Therefore, although not particularly limited, for example, the dose for a human (preferably an adult) is preferably 0.0001 to 10 mg, and more preferably 0.001 to 5 mg, per kg of the subject's body weight and per day, in terms of the amount of NMN. The number of administrations can be divided from once a day to an appropriate number of times.

### [Examples]

Hereinafter, the present invention will be described more specifically based on Test Examples, but the present invention is not limited to the following examples. In the following Test Examples, the notation "%" indicates weight/volume (w/v: g/mL) percent, unless otherwise specified.

### <LC-MS/MS Analysis>

In each of the following Test Examples, the measurement of NMN concentration (ng/mL) and NAD⁺ concentration (ng/mL) was performed by LC-MS/MS analysis. The LC-MS/MS analysis was performed under the following conditions:
Column: Hypercarb 3 µm, 2.1 mm x 100 mm Column
Mobile phase:
   Solution A: 7.5 mM ammonium acetate aqueous solution containing 0.05 v/v% ammonium hydroxide
   Solution B: Acetonitrile solution containing 0.05 v/v% ammonium hydroxide
   Ratio of solution A and solution B in the mobile phase:
      Start of mobile phase flow 4 0 min: Solution A 95 v/v% - Solution B 5 v/v%
      0 min → 1.8 min: Solution A 95 v/v% - Solution B 5 v/v%
      1.8 min → 14 min: Solution A 95 v/v% - Solution B 5 v/v% → Solution A 46 v/v% - Solution B 54 v/v%
      14 min → 14.1 min: Solution A 46 v/v% - Solution B 54 v/v% → Solution A 10 v/v% - Solution B 90 v/v%
      14.1 min → 17.1 min: Solution A 10 v/v% - Solution B 90 v/v%
      17.1 min → 17.2 min: Solution A 95 v/v% - Solution B 5 v/v%
      17.2 min → 32.2 min: Solution A 95 v/v% - Solution B 5 v/v%
Column temperature: 60°C
Flow rate: 0.2 mL/min
Injection volume: 2 µL
Ionization: ESI positive mode
m/z of NMN:
   Precursor ion (Q1 Mass, (Da)): 335.1
   Product ion (Q3 Mass, (Da)): 123.0 m/z of NAD⁺:
      Precursor ion (Q1 Mass, (Da)): 664.2
      Product ion (Q3 Mass, (Da)): 428.2

### <Test Example 1>

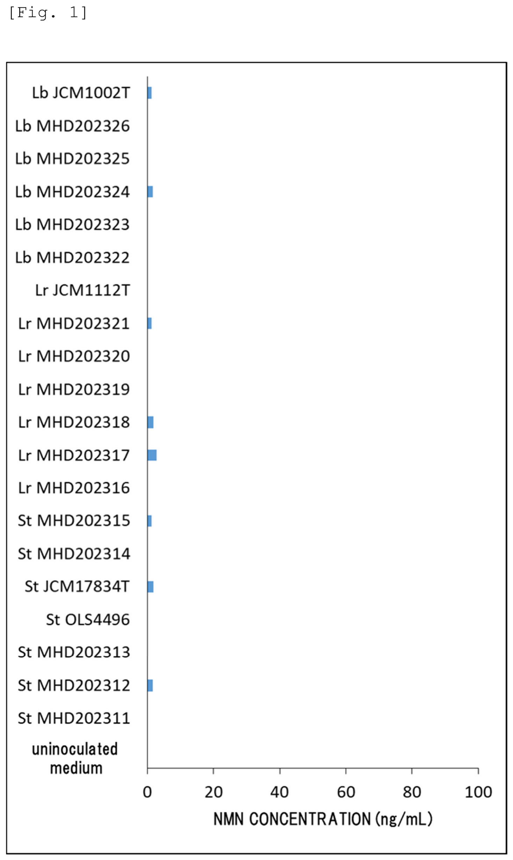

First, each bacterial strain of Lactobacillus delbrueckii, Limosilactobacillus reuteri, and Streptococcus thermophilus shown in Table 1 below was subjected to activation culture twice in SMY medium at 37°C for 18 hours under anaerobic conditions using an AnaeroPack. The SMY medium is a medium prepared by dissolving 10 w/w% of skim milk powder (manufactured by Meiji Co., Ltd.) and 0.1 w/w% of yeast extract (manufactured by Difco) in water, and then autoclave sterilizing at 115°C for 15 minutes (non-fat milk solids content: 9.55 w/w%). Next, each culture solution after activation culture was inoculated into fresh SMY medium such that the concentration was 1 v/v% (1 × 10⁶ to 1 × 10⁸ cfu/g), and was anaerobically cultured at 37°C for 12 hours. A 20 µL sample of the culture solution after culturing was taken, 200 µL of 88.8 v/v% methanol was added, and after mixing well, the mixture was allowed to stand on ice for 30 minutes. After standing, the mixture was centrifuged at 15,000 rpm at 4°C for 2 minutes to collect 170 µL of the supernatant, which was then dried to a solid using a centrifugal evaporator. The dried product was dissolved in a 10 mM ammonium acetate solution and then filtered through a 0.22 µm diameter filter, and the filtrate was subjected to LC-MS/MS to measure the NMN concentration (ng/mL) in the SMY medium after culturing. The results (SMY) are shown in Table 1 and Fig. 1 below. In the following tables and figures, "St" is an abbreviation for "Streptococcus thermophilus," "Lr" is for "Limosilactobacillus reuteri," and "Lb" is for "Lactobacillus delbrueckii ssp. bulgaricus," respectively, and "0" indicates that NMN was not detected or was below the detection limit.

### <Test Example 2>

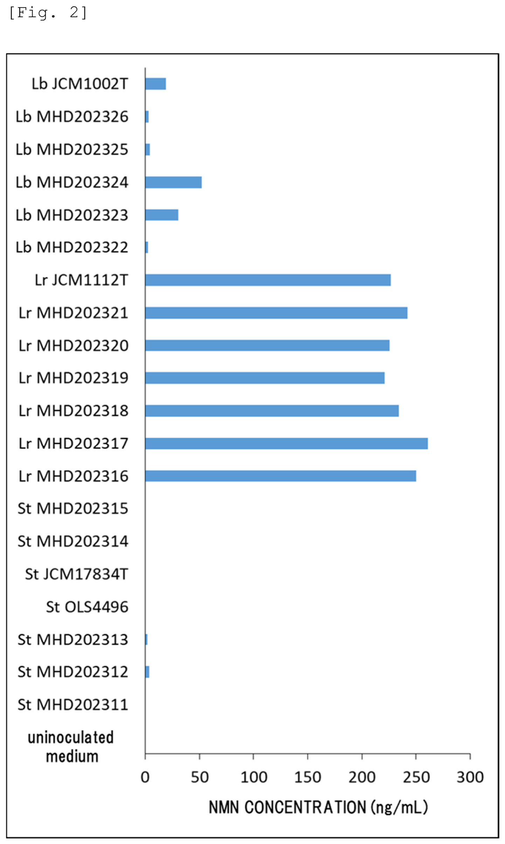

Each bacterial strain of Lactobacillus delbrueckii, Limosilactobacillus reuteri, and Streptococcus thermophilus was cultured in the same manner as in Test Example 1, except that an SMY+NAD medium was used instead of the SMY medium as the medium into which the culture solution after each activation culture was inoculated, and the NMN concentration (ng/mL) in the SMY+NAD medium after culturing was measured. The SMY+NAD medium is a medium prepared in the same manner as the SMY medium, except that NAD⁺ (manufactured by Sigma-Aldrich) was further added to the SMY medium after autoclave sterilization to a final concentration of 5,000 ng/mL. The results (SMY+NAD) are shown in Table 1 and Fig. 2 below.

**[Table 1]**

| | | SMY | SMY+NAD |
|---|---|---|---|
| Uninoculated medium | | 0 | 0 |
| Streptococcus thermophilus | St MHD202311 | 0 | 0 |
| | St MHD202312 | 2 | 4 |
| | St MHD202313 | 0 | 2 |
| | St OLS4496 | 0 | 0 |
| | St JCM17834T | 2 | 0 |
| | St MHD202314 | 0 | 0 |
| | St MHD202315 | 1 | 0 |
| Limosilactobacillus reuteri | Lr MHD202316 | 0 | 250 |
| | Lr MHD202317 | 3 | 261 |
| | Lr MHD202318 | 2 | 234 |
| | Lr MHD202319 | 0 | 221 |
| | Lr MHD202320 | 0 | 225 |
| | Lr MHD202321 | 1 | 242 |
| | Lr JCM1112T | 0 | 227 |
| Lactobacillus delbrueckii ssp. bulgaricus | Lb MHD202322 | 0 | 2 |
| | Lb MHD202323 | 0 | 30 |
| | Lb MHD202324 | 2 | 52 |
| | Lb MHD202325 | 0 | 4 |
| | Lb MHD202326 | 0 | 3 |
| | Lb JCM1002T | 1 | 19 |

| | | | |
|---|---|---|---|
| NMN concentration (ng/mL) | | | |

As shown in Table 1 and Fig. 1, it was confirmed that in SMY medium, some strains of Lactobacillus delbrueckii and Limosilactobacillus reuteri produced NMN, but the amount thereof was small. On the other hand, as shown in Table 1 and Fig. 2, by adding NAD⁺ to the SMY medium, it was confirmed that even Lactobacillus delbrueckii and Limosilactobacillus reuteri that did not produce NMN in SMY medium alone became able to produce NMN, and it was further confirmed that the production amount thereof also increased significantly. Meanwhile, Streptococcus thermophilus either did not produce NMN or produced almost none in any of the strains and media. The cultures of Lactobacillus delbrueckii and Limosilactobacillus reuteri shown in Table 1 all exhibited yogurt-like physical properties.

### <Test Example 3>

An improvement in the NMN production amount by the addition of NAD⁺-producing Streptococcus thermophilus, instead of the addition of NAD⁺, was verified. The NAD⁺ concentrations (ng/mL) in the SMY medium measured after the culture of Test Example 1 for the Streptococcus thermophilus used in the following tests are shown in Table 2 below. As shown in Table 2, all Streptococcus thermophilus strains produced a sufficient amount of NAD⁺ in the SMY medium.

**[Table 2]**

| Uninoculated medium | 0 |
|---|---|
| St MHD202311 | 4009 |
| St MHD202312 | 5039 |
| St MHD202313 | 3986 |

| | |
|---|---|
| NAD+ Concentration (ng/mL) | |

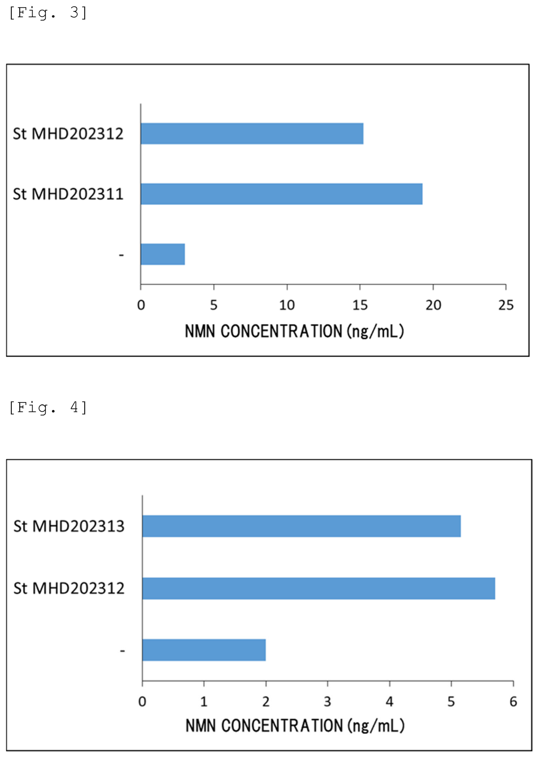

First, each bacterial strain of Lactobacillus delbrueckii, Limosilactobacillus reuteri, and Streptococcus thermophilus shown in Tables 3 to 4 below was subjected to activation culture twice in the SMY medium at 37°C for 18 hours under anaerobic conditions using an AnaeroPack. Next, into fresh SMY medium, in the combinations shown in Tables 3 to 4 below, the culture solutions after activation culture of each bacterial strain were inoculated such that the concentration was 1 v/v% (1 × 10⁶ to 1 × 10⁸ cfu/g) for each strain, and were anaerobically cultured at 37°C for 12 hours. The culture solution after culturing was subjected to LC-MS/MS in the same manner as in Test Example 1, and the NMN concentration (ng/mL) in the SMY medium after culturing was measured. The results are shown in Tables 3 to 4 and Figs. 3 to 4 below.

**[Table 3]**

| | Lr MHD202317 |
|---|---|
| - | 3 |
| St MHD202311 | 19.3 |
| St MHD202312 | 15.2 |

| | |
|---|---|
| NMN Concentration (ng/mL) | |

**[Table 4]**

| | Lb MHD202324 |
|---|---|
| - | 2 |
| St MHD202312 | 5.7 |
| St MHD202313 | 5.2 |

| | |
|---|---|
| NMN Concentration (ng/mL) | |

As shown in Tables 3 to 4 and Figs. 3 to 4, with only Lactobacillus delbrueckii or Limosilactobacillus reuteri ("-" in the tables and figures), the NMN production amount was small, but it was confirmed that by the addition of NAD⁺-producing Streptococcus thermophilus, the NMN production amount by these Lactobacillus delbrueckii and Limosilactobacillus reuteri also increased, similar to the results of Test Example 2. The cultures with Limosilactobacillus reuteri and Streptococcus thermophilus shown in Table 3 and the cultures with Lactobacillus delbrueckii and Streptococcus thermophilus shown in Table 4 all exhibited yogurt-like physical properties.

### <Test Example 4>

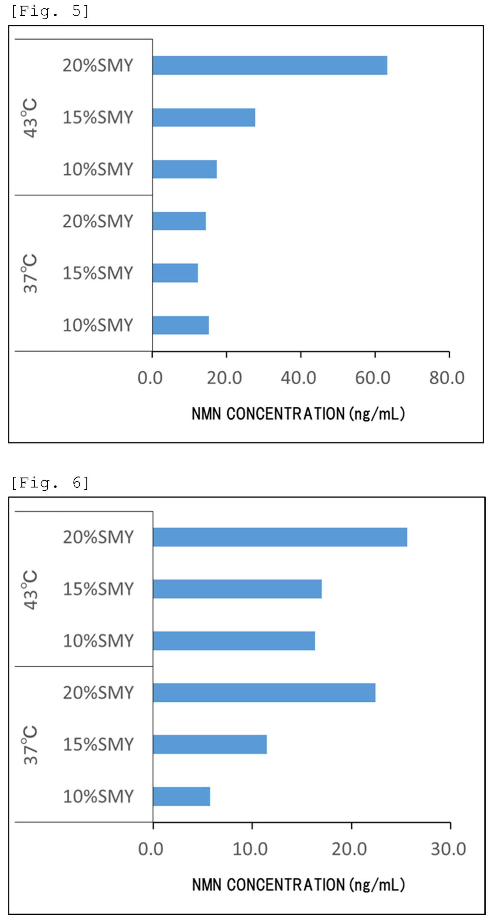

Suitable temperature and medium conditions for NMN production by the addition of NAD⁺ as confirmed above were verified. First, in the SMY medium, SMY media of each concentration (10% SMY, 15% SMY, 20% SMY) were prepared in the same manner except that the concentration of the skim milk powder was set to 10 w/w% (non-fat milk solids concentration: 9.55 w/w%), 15 w/w% (non-fat milk solids concentration: 14.33 w/w%), or 20 w/w% (non-fat milk solids concentration: 19.10 w/w%). Using these SMY media, the NMN concentration (ng/mL) in the SMY medium after culturing was measured in the same manner as in Test Example 3, except that the combination of bacterial strains was a combination of Lr MHD202317 and St MHD202312, or a combination of Lb MHD202324 and St MHD202312, and the culture temperature was 37°C or 43°C. The results are shown in Table 5 and Figs. 5 to 6 below. As shown in Table 5 and Figs. 5 to 6, it was confirmed that in both combinations, the NMN production amount was highest at a skim milk powder concentration of 20 w/w% and a culture temperature of 43°C.

**[Table 5]**

| | | Lr MHD202317 | Lb MHD202324 |
|---|---|---|---|
| | | St MHD202312 | St MHD202312 |
| 37°C | 10%SMY | 15.2 | 5.7 |
| | 15%SMY | 12.2 | 11.5 |
| | 20%SMY | 14.4 | 22.4 |
| 43°C | 10%SMY | 17.4 | 16.3 |
| | 15%SMY | 27.7 | 17.0 |
| | 20%SMY | 63.3 | 25.6 |

| | | | |
|---|---|---|---|
| NMN Concentration (ng/mL) | | | |

### <Test Example 5>

First, 10 kg of skim milk powder (manufactured by Meiji Co., Ltd.), 0.1 kg of yeast extract (manufactured by Asahi Group Foods, Ltd.), and 89.9 kg of water were mixed, heated until it reached a temperature of 95°C for heat sterilization, and then cooled to 43°C. Next, 0.1 kg of a lactic acid bacterium starter (Lr MHD202317 and St MHD202312) was inoculated, filled into containers, and subjected to static fermentation in a constant temperature room at 43°C to obtain a yogurt-like composition. When this composition was subjected to LC-MS/MS in the same manner as in Test Example 1, the NMN concentration in this composition was measured and found to be 17.4 ng/mL.

### <Test Example 6>

First, 10 kg of skim milk powder (manufactured by Meiji Co., Ltd.), 0.1 kg of yeast extract (manufactured by Asahi Group Foods, Ltd.), and 89.9 kg of water were mixed, heated until it reached a temperature of 95°C for heat sterilization, and then cooled to 43°C. Next, 0.1 kg of a lactic acid bacterium starter (Lb MHD202324 and St MHD202312) was inoculated, filled into containers, and subjected to static fermentation in a constant temperature room at 43°C to obtain a yogurt-like composition. When this composition was subjected to LC-MS/MS in the same manner as in Test Example 1, the NMN concentration in this composition was measured and found to be 16.3 ng/mL.

### <Test Example 7>

First, 10 kg of skim milk powder (manufactured by Meiji Co., Ltd.), 0.1 kg of yeast extract (manufactured by Asahi Group Foods, Ltd.), and 89.9 kg of water were mixed, heated until it reached a temperature of 95°C for heat sterilization, and then cooled to 43°C. Next, 1 g of NAD⁺ (manufactured by Sigma-Aldrich), 0.1 kg of a lactic acid bacterium starter (Lb MHD202324 and St MHD202312), and 0.1 kg of Lr MHD202317 were inoculated, filled into containers, and subjected to static fermentation in a constant temperature room at 43°C to obtain a yogurt-like composition. When this composition was subjected to LC-MS/MS in the same manner as in Test Example 1, the NMN concentration in this composition was measured and found to be 260 ng/mL.

### [Industrial Applicability]

As explained above, according to the present invention, it becomes possible to provide a method for producing yogurt that enables the production of yogurt containing a sufficient amount of nicotinamide mononucleotide (NMN) using Lactobacillus delbrueckii and Limosilactobacillus reuteri. Furthermore, according to the present invention, it also becomes possible to provide an NMN production method capable of producing a sufficient amount of NMN, and an NMN production amount improvement method capable of improving the amount of NMN production by the lactic acid bacteria.

### [Accession Number]

1.
   (1) Identification: Streptococcus thermophilus OLS4496
   (2) Accession Number: NITE BP-02875
   (3) Date of Deposit: February 5, 2019
   (4) Depositary Institution: NITE Patent Microorganisms Depositary (NPMD), National Institute of Technology and Evaluation.

## Claims

1. A method for producing yogurt, comprising a culturing step of culturing at least one NMN-producing lactic acid bacterium selected from the group consisting of Lactobacillus delbrueckii and Limosilactobacillus reuteri in an NAD-containing medium prepared by adding nicotinamide adenine dinucleotide to a milk component-containing medium, and obtaining a yogurt containing nicotinamide mononucleotide.

2. The method for producing yogurt according to claim 1, wherein a content of nicotinamide mononucleotide derived from the NAD-containing medium and the lactic acid bacterium in the yogurt is 5 ng/mL or more.

3. The method for producing yogurt according to claim 1, wherein a content of milk components in the milk component-containing medium is 5 to 35 w/w% in terms of a content of non-fat milk solids.

4. The method for producing yogurt according to claim 1, wherein a temperature in the culturing is 25 to 55°C.

5. The method for producing yogurt according to claim 1, wherein a content of nicotinamide adenine dinucleotide in the NAD-containing medium is 1 × 10² to 1 × 10⁸ ng/mL relative to a total amount of the NAD-containing medium.

6. The method for producing yogurt according to claim 1, wherein the addition of nicotinamide adenine dinucleotide to the medium is the addition of Streptococcus thermophilus, which produces nicotinamide adenine dinucleotide in the medium.
